# EUROPEAN PATENT APPLICATION

(11) **EP 4 338 653 A1**
(43) Date of publication of application: **20.03.2024**
(21) Application number: 22196381.2
(22) Date of filing: 19.09.2022
(51) Int. Cl.: A61B 1/00, A61B 1/05, A61B 1/06, A61B 16/00, A61B 18/00, A61B 18/20, A61B 18/22, G01N 1/28, G01N 1/44, G06T 7/55, G16H 30/40, G01N 1/32, G01N 1/36

(54) **APPARATUS AND METHOD FOR FACILITATING A THREE-DIMENSIONAL HISTOLOGICAL RECONSTRUCTION**

(71) Applicant: Universitätsklinikum Hamburg-Eppendorf (UKE), 20246 Hamburg (DE)
(72) Inventor: Schmitz, Rüdiger, 20251 Hamburg (DE); Steurer, Stefan, 20251 Hamburg (DE); Hahn, Jan, 20357 Hamburg (DE)
(74) Representative: Eisenführ Speiser

(57) **Abstract**

The invention relates to an apparatus 100 for facilitating a three-dimensional histological reconstruction. The apparatus 100 comprises a reconstruction pattern application device 109-115 configured to apply a reconstruction pattern to a surface of a histological specimen 10, wherein the surface will be decomposed along slice contours arising from a predefined histological slicing to be carried out on the specimen 10. The applied reconstruction pattern intersects the slice contours so as to be decomposed by the histological slicing and restored upon three-dimensional histological reconstruction.

## Description

### FIELD OF THE INVENTION

The invention relates to an apparatus, a method and a computer program for facilitating a three-dimensional histological reconstruction, as well as to a corresponding histological specimen and corresponding histological slices. Furthermore, the invention relates to a system, a method and a computer program for three-dimensional histological reconstruction. The invention also relates to an apparatus for marking specimen tissue to be extracted from an anatomical region in a subject's body, and to a use thereof.

### BACKGROUND OF THE INVENTION

A histopathological workup is considered the gold standard and backbone of medical diagnostics for many diseases, amongst them cancer. This workup routine comprises a multistep process: The tissue specimen is first extracted from a subject's body by means of a surgical or interventional procedure. This results in one or more specimens, depending on the extraction procedure. Second, the one or more specimens are, optionally, divided into yet smaller pieces before the actual histopathological workup. For the latter, the one or more specimens are then, third, sliced into a plurality of histopathological slices. These slices are then, fourth, stained, and, fifth, analysed microscopically. This can provide deep insights into the tissue structure. However, due to the surgical extraction (first step), the optional partitioning of the extracted specimen before the actual histopathological workup (second step), and the slicing of the one or more specimens (third step), information regarding a spatial relation of the histological slices with respect to each other as well as, collectively, with respect to their anatomical origin can be lost.

Three-dimensional histological reconstruction is an attempt to recover at least the information regarding the spatial relation of the histological slices with respect to each other, thereby effectively registering the histological slices. In order to facilitate a three-dimensional histological reconstruction of a histological specimen, the specimen can be provided, for instance, with fiducial marks that can be detected in the histological slices in order to use them for registration. In particular, as described in the article "Robust Alignment of Prostate Histology Slices With Quantified Accuracy" by C. Hughes et al., IEEE Transactions on Biomedical Engineering, volume 60, number 2 (2013), needles can be inserted into the specimen to thereby create internal marks in the form of holes visible inside the tissue of the histological slices. However, this approach introduces serious damage to the tissue, which in itself is undesirable, and also requires the marks to be applied with a certain safety margin from anatomical points of interest, such as tumours, thereby rendering the marking, if at all applicable, less precise. Moreover, this approach is restricted to tissue samples that are mechanically stable enough to be punctured in a direction of the histological slicing, i.e. the direction perpendicular to the later sectioning planes.

There is therefore a need for less invasive means for facilitating a three-dimensional histological reconstruction.

### SUMMARY OF THE INVENTION

It is an object of the invention to provide less invasive means for facilitating a three-dimensional histological reconstruction.

The above-mentioned object is achieved by an apparatus for facilitating a three-dimensional histological reconstruction, wherein the apparatus comprises a reconstruction pattern application device configured to apply a reconstruction pattern to a surface of a histological specimen, wherein the surface will be decomposed along slice contours arising from a predefined histological slicing to be carried out on the specimen, wherein the applied reconstruction pattern intersects the slice contours so as to be decomposed by the histological slicing and restored upon three-dimensional histological reconstruction.

Since the reconstruction pattern is applied to a surface of the histological specimen that will be decomposed along slice contours arising from a predefined histological slicing to be carried out on the specimen, wherein the applied reconstruction pattern intersects the slice contours so as to be decomposed by the histological slicing and restored upon three-dimensional histological reconstruction, a sufficiently precise reconstruction pattern can be applied while causing only relatively little damage to the specimen. A less invasive three-dimensional histological reconstruction can hence be achieved. It has been found that the relatively small degree of damage caused to the specimen, which translates to the histological slices generated therefrom, also allows for a more accurate histological analysis. Moreover, it has been found that, since, as opposed to, for instance, generating internal marks in the bulk of the specimen by inserting a needle, the reconstruction pattern is applied to the surface of the specimen such that it intersects the slice contours arising from the predefined histological slicing, a three-dimensional reconstruction is facilitated even in case the histological slicing is to be carried out on relatively thin tissue, specifically if this is to be sliced in a direction such that the thinness translates to the histological slices. In particular, also a three-dimensional histological reconstruction of predominantly mucosal tissue specimens is facilitated.

The surface of the specimen on which the reconstruction pattern is applied can be, for instance, a natural tissue surface, a surgical resection surface, or a surface generated by cutting an originally extracted specimen subsequently, i.e. after resection, into smaller specimen parts, i.e. an ex-vivo, periprocedurally and newly created surface. For hygienic reasons, the apparatus may comprise an air sealing under which the specimen can be placed.

Carrying out the histological slicing after the reconstruction pattern has been applied to the surface will typically break up the reconstruction pattern into a plurality of edge marks present on edges of slices generated by the histological slicing. The three-dimensional histological reconstruction may then amount to a restauration, or recomposition, of the reconstruction pattern from the plurality of edge marks. For instance, a line in the reconstruction pattern would, if the reconstruction pattern is cut or ablated into the surface of the specimen, be broken up into edge marks in the form of characteristic recesses, or notches, in an outer tissue contour of the histological slices.

The reconstruction pattern is preferably chosen such that a spatial relation between the histological slices of the specimen arising from the histological slicing can be analytically deduced from the edge marks. A "spatial relation" between the histological slices can particularly refer to a relative position, wherein the term "position" is understood herein as including both a location and an orientation. Hence, the spatial relation between two given histological slices may, for instance, be defined in terms of their relative distance and their relative orientation.

It can be advantageous if the reconstruction pattern is chosen such that it intersects each slice contour on the surface of the specimen more than once, i.e. a plurality of times. A spatial relation between the histological slices can then be determined based on the plurality of intersections with an increased precision, particularly including information on a spatial precision of the histological reconstruction, such as given, for instance, by residual errors.

The reconstruction pattern can be fixed. In this case, the apparatus will, by means of the reconstruction pattern application device, always apply the same reconstruction pattern. This can suffice for many cases, particularly for specimens have a similar shape and size.

In contrast, the apparatus may also comprise a reconstruction pattern determining unit configured to determine the reconstruction pattern to be applied to the surface of the histological specimen based on a user input and/or one or more determining factors. For instance, the reconstruction pattern determining unit may be configured to determine the reconstruction pattern to be applied to the surface of the histological specimen based on the predefined histological slicing, i.e., based on a predefined slicing pattern. This makes the apparatus more flexible in use. For instance, the reconstruction pattern may be determined based on a slicing direction predefined for the histological slicing to be carried out. A "slicing direction" is understood herein as a direction perpendicular to which one or more, and typically all, histological slices will be cut out of the specimen. The reconstruction pattern determining unit may be configured to determine the reconstruction pattern to comprise a plurality of lines, wherein each of the lines extends in a direction that has a nonzero component in the slicing direction. It will be understood that a line in a direction perpendicular to the slicing direction would lie in, or parallel to, one of the histological slicing planes and would therefore not intersect with any of the slice contours on the surface of the specimen. Such a line is therefore preferably not chosen for the reconstruction pattern.

The apparatus may further comprise an image data providing unit configured to provide image data for a two-dimensional image of the histological specimen, wherein the image corresponds to a view onto the surface of the specimen that will be decomposed by the histological slicing. The image, or the image data, may be acquired using an imaging device like a camera, for instance. The reconstruction pattern determining unit may be configured to determine the reconstruction pattern to be applied to the surface of the histological specimen based further on the image data. This allows for a further flexibility in applying the reconstruction pattern. In particular, the reconstruction pattern determining unit may be configured to determine the reconstruction pattern based further on the histological specimen as seen in the two-dimensional image. For instance, the reconstruction pattern determining unit may be configured to overlay the slice contours that will arise from the predefined histological slicing on the surface of the specimen in the two-dimensional image, and determine the reconstruction pattern to be applied to the surface based on the overlay including the slice contours. In this way, the reconstruction pattern may be defined with respect to the surface of the specimen as seen in the image for which the image data are provided.

For instance, the reconstruction pattern may be overlaid on the surface of the specimen in the image together with the slice contours. Even if the apparatus further comprises a display, which may be the case, the overlay is not necessarily displayed, but may be used for controlling the reconstruction pattern application device in applying the reconstruction pattern to the surface. Additionally or alternatively, the reconstruction pattern determining unit may be configured to determine a boundary of the surface of the specimen in the two-dimensional image based on the provided image data and to determine the reconstruction pattern based on the determined boundary.

A determination of the reconstruction pattern based on the boundary of the surface of the histological specimen allows to choose an optimal reconstruction pattern depending on a shape of the surface. For instance, a basic reconstruction pattern may be scaled up or down such that it covers the specimen surface on which it is to be applied entirely.

The reconstruction pattern may be a line pattern. The line pattern may consist of one or more lines, wherein the lines are preferably straight lines. An exemplary reconstruction pattern comprises two parallel lines and a third line crossing the at least two parallel lines. It has been found that such a relatively simple reconstruction patterns already allow for a robust determination of the orientation and location of the sectioning planes in which the histological slices are taken, and hence of the spatial relation between the histological slices. The one or more lines are preferably scaled so as to extend over the surface of the specimen in at least a portion of the surface on which the specimen is to be sliced.

For a partial positioning of the histological slices, also a reconstruction pattern comprising just two or more parallel lines may be sufficient. On the other hand, geometrically more complex reconstruction patterns may be used. For instance, star-shaped reconstruction patterns can be applied, which may allow for a better distinguishability between inner and outer regions of the specimen surface.

It may be preferred that, instead of only a single reconstruction pattern, a plurality of reconstruction patterns are determined and thereafter applied to one or more surfaces of the specimen. However, it will be understood that a plurality of reconstruction patterns could equally be understood as a single reconstruction pattern, possibly of a more complex nature. A single reconstruction pattern may, for instance, comprise a plurality of reconstruction subpatterns, wherein the subpatterns may be different versions of one and the same basic reconstruction pattern. Applying more than one reconstruction pattern can increase the quality of reconstruction, particularly if the respective specimen is a non-flat specimen and/or comprises more than one surface.

When one and the same reconstruction pattern or subpattern is repeated more than once over one or more surfaces of the histological specimen or in different regions of one and the same, possibly curved, specimen surface, it can become difficult to distinguish the different instances of one and the same reconstruction pattern or subpattern from each other. Therefore, it may be preferred that repeating elements in the one or more reconstruction patterns are provided with distinguishing features. By providing repeating elements in the determined one or more reconstruction patterns with distinguishing features, regular reconstruction patterns can be used without risking to confuse like elements of the patterns with each other and thereby cause reconstruction errors.

For instance, when applying a reconstruction pattern consisting of two parallel lines and a third line crossing the two parallel lines twice to different surfaces of the specimen, thereby creating two copies of one and the same reconstruction pattern, a first of the copies may be applied such that the respective three lines have a first line width, and the second of the two copies may be applied such that the respective three lines are applied with a second line width, wherein the first and the second line width are different from each other. In this way, the edge marks appearing in the histological slices will either have the first or the second width, wherein it can be deduced therefrom to which of the two copies of the reconstruction pattern the edge marks belong.

Instead of providing the two copies of the reconstruction pattern with different line widths, other distinguishing features can be chosen, such as a multiplicity of the lines or a depth of the lines, for instance. A line "multiplicity" refers herein to a number of sublines making up a main line of the reconstruction pattern. For instance, each line of a reconstruction pattern may actually consist of two, three or more finer, parallel sublines. Additionally or alternatively, further reconstruction patterns may also be applied in a different orientation than previous reconstruction patterns.

It may, furthermore, be preferred that the image data providing unit is configured to provide image data for a plurality of two-dimensional images of the histological specimen, wherein each of the images corresponds to a different view onto the surface of the specimen, wherein the reconstruction pattern determining unit is configured to determine the reconstruction pattern with respect to each of the images. In this way, a three-dimensional histological reconstruction is facilitated even for non-flat and other specimens with a more complex geometry. Also, determining a full three-dimensional position of the individual histological slices is facilitated more robustly in this way. For instance, the reconstruction pattern may comprise a front part and a back part, wherein the front part is to be applied to a front surface of the specimen and the back part is to be applied to a back surface of the specimen. The front surface and the back surface are understood as opposite sides of the same surface of the specimen.

The reconstruction pattern application device may be configured to apply the reconstruction pattern to the surface of the specimen by any of the following: mechanical cutting, laser cutting, inking, electro-cauterisation, coagulation, particularly argon-plasma coagulation, any combination of the foregoing.

The reconstruction pattern application device may be automated. For instance, laser cutting may be automated by moving the laser while the specimen is fixed, by moving the specimen (such as, for instance, using a mechanical, "x-y", table) while the laser is fixed, or by a combination of the foregoing, i.e. by moving both the laser and the specimen at least temporarily. The reconstruction pattern application device may also be configured to apply the reconstruction pattern by automated inking, such as, for instance, by automatically spraying lines or colour gradients onto the respective surface of the specimen. Likewise, electro-cauterisation and related techniques like argon-plasma coagulation can be automated by the reconstruction pattern application device to apply the reconstruction pattern. However, the reconstruction pattern application device may also be configured to allow for a manual or semi-automatic application of the reconstruction pattern. A suitable user interface may be provided for this purpose. A laser of the reconstruction pattern application device could even be configured for handheld use.

Whether automated or not, to allow for direct marking on the specimen surface - the region of interest to the pathologist - it is of vital importance that diagnostic quality is not affected. To this end, it may be preferred that a laser system is chosen that is able to ablate tissue whilst leaving the neighbouring, remaining tissue as much unaffected as possible. According to a particularly preferred embodiment, the reconstruction pattern application device comprises a laser, especially a pulsed infrared laser, for applying the reconstruction pattern to the surface of the specimen. In this way, e.g. cold vaporisation can be effected in the tissue along the reconstruction pattern, which saves neighbouring tissue from damage. Particularly micro-, nano- or picosecond infrared lasers can be used. The laser preferentially is a laser which does not damage or does only a little damage the tissue below or beside the applied reconstruction pattern. For this reason, the laser preferentially is a pulsed infrared laser, but it also can be another type of laser, especially another type of laser effecting cold vaporisation. Generally, the laser preferentially is a laser with the property that tissue can be precisely ablated and minimum damage is introduced to the non-ablated tissue.

The term "cold vaporisation" is used to refer to processes in which a decomposition of tissue can be achieved without a significant, particularly without a dangerous amount of, transfer of thermal energy into the tissue, i.e. regions surrounding the decomposed tissue region. It has been found that, by tuning an infrared laser such that its energy is absorbed by water molecules and immediately converted into translational energy caused by the vibrational motion of their OH stretch band, an explosion of the irradiated water molecules can be achieved, which leads to a decomposition of tissue and thereby a transfer into the gas phase. In the case of a picosecond infrared laser (PIRL), the transfer of the energy of the laser into translational energy is much faster than the transfer into thermal energy, so that tissue irradiated with a picosecond infrared laser is transferred into an aerosol by cold vaporisation. However, it has been found that also, for instance, micro- or nanosecond infrared lasers can be used for effecting cold vaporisation. A particularly preferred wavelength to effect cold vaporisation is 2.94 µm. However, also other wavelengths, particularly in the infrared range, are possible.

Preferably, the apparatus comprises an imaging device for imaging the applied reconstruction pattern on the surface of the specimen, i.e. the reconstruction pattern as applied by the reconstruction pattern application device to the specimen. This allows to verify whether the reconstruction pattern has been accurately applied by the reconstruction pattern application device. The imaging device used for this purpose is preferably different from any imaging device used for imaging the specimen surface in order to, for instance, extract a specimen surface boundary therefrom. An exemplary imaging device that can be used for the purpose of verifying the pattern application is an optical coherence tomography (OCT) device. Optical coherence tomography allows for an accurate verification of a degree to which the intended reconstruction pattern has been correctly applied to the specimen surface.

If the imaging device, i.e. the imaging device used for the "verification" purpose, is used to image the applied reconstruction pattern during the application process, the application of the reconstruction pattern can be modified and/or complemented once an inaccurate or even wrong application of a part of the reconstruction pattern has been detected. For instance, an error detection unit of the apparatus may be configured to detect a mismatch between an intended reconstruction pattern and an applied reconstruction pattern based on one or more images provided by the imaging device. If a detected mismatch exceeds a predetermined threshold, for instance, additional lines of a line pattern corresponding to the reconstruction pattern may be applied by the reconstruction pattern application device. These additional lines may be applied with a different depth and/or multiplicity as compared to the original reconstruction pattern, which has been improperly applied.

Instead of correcting for an inaccurately applied reconstruction pattern while still applying the reconstruction pattern, or in fact additionally to doing so, any inaccuracy in the application of the reconstruction pattern may also be accounted for in the reconstruction process. For instance, a three-dimensional reconstruction of the histological specimen from the histological slices may be carried out based on one or more images of the applied reconstruction pattern, particularly based on any detected non-parallelity of lines in a reconstruction pattern or a mismatch in an angle formed by two lines between a) the reconstruction pattern as intended and b) the applied reconstruction pattern.

In an example, a reconstruction pattern corresponding to two parallel lines having a certain intended distance to each other may be applied to the surface of the specimen. In order to deduce the orientation of a histological slice whose slice contour intersects the two parallel lines with respect to these lines, the intended distance between the two parallel lines is compared with a distance of the two edge marks left by the two parallel lines on the edge of the histological slice. If, now, the actual distance between the lines deviates from the intended distance, the deduced orientation of the histological slice will be incorrect. By measuring the actual distance between the two parallel lines of the reconstruction pattern using, for instance, optical coherence tomography, such errors can be avoided.

It should be noted that, instead of using an automatic reconstruction pattern application device, the reconstruction pattern may also be applied manually. Also, a manual application of the reconstruction pattern to the surface of the specimen can be carried out by any of: mechanical cutting, laser cutting, inking, electro-cauterisation, coagulation, particularly argon-plasma coagulation, any combination of the foregoing. For a manual application of the reconstruction pattern, a visualisation device configured to visualise the reconstruction pattern on the surface of the specimen for a user applying the reconstruction pattern may be used. For instance, the reconstruction pattern may be projected onto the surface of the specimen, wherein the user may then cut, ablate, cauterise, illuminate, etc. the surface of the specimen according to the projected reconstruction pattern.

The invention has so far been described with regard to an apparatus for facilitating a three-dimensional histological reconstruction by applying a reconstruction pattern to a surface of a histological specimen that will be decomposed along slice contours arising from a predefined histological slicing to be carried out on the specimen. However, it will be understood that the three-dimensional histological reconstruction actually does not rely on the apparatus itself, but on the applied reconstruction pattern.

Hence, in a further aspect, a histological specimen is presented that comprises a surface that will be decomposed along slice contours arising from a predefined histological slicing to be carried out on the specimen, wherein the surface comprises a reconstruction pattern applied to it that intersects the slice contours so as to be decomposed by the histological slicing and restored upon three-dimensional histological reconstruction. Such a specimen may be, but is not necessarily, obtained using an apparatus as described above. The predefined histological slicing can be carried out on it without risking to lose the information about the spatial relationship between the histological slices, since the reconstruction pattern has been applied to the specimen in the specified manner.

According to a related aspect, the specimen of the previous aspect is presented in its sliced form. Hence, a plurality of histological slices arisen form a histological slicing carried out on a histological specimen is presented, wherein the histological slicing has been carried out such that a surface of the specimen has been decomposed along slice contours intersecting a reconstruction pattern applied to the specimen before carrying out the slicing and decomposed into edge marks of the histological slices, wherein the histological slices are recomposable such that the edge marks reform the reconstruction pattern on the surface of the recomposed specimen.

In a further aspect, a system for three-dimensional histological reconstruction is presented. The system comprises a reconstruction pattern providing unit configured to provide data indicative of a reconstruction pattern that has been applied to a surface of a histological specimen, a slice image data providing unit configured to provide image data for two-dimensional images of histological tissue slices generated from the histological specimen by a decomposition of the surface to which the reconstruction pattern has been applied along slice contours intersecting the reconstruction pattern, and a reconstruction unit. The reconstruction unit is configured to reconstruct three-dimensional image data for a volumetric image of the histological specimen based on the data indicative of the applied reconstruction pattern and the image data for the two-dimensional images of the histological slices such that, in the volumetric image, the reconstruction pattern is restored on the surface of the histological specimen. Hence, the system builds upon a reconstruction pattern being applied to a surface of the specimen as discussed with respect to the previous aspects. Preferably, the reconstruction pattern has been applied using the apparatus comprising the reconstruction pattern application device described above.

The data indicative of the applied reconstruction pattern which are provided by the reconstruction pattern providing unit may refer to geometric parameters of the reconstruction pattern. For instance, in case the reconstruction pattern is a line pattern comprises two or more parallel lines and one or more further lines angulated with respect to the parallel lines, the geometric parameters may comprise one or more inter-parallel line distances corresponding to the one or more distances between the parallel lines, and one or more angles indicating a respective angle at which the one or more angulated lines intersect the parallel lines. The data indicative of the applied reconstruction pattern may be image or image-inferred data. In particular, the data indicative of the applied reconstruction pattern could be determined by the reconstruction pattern providing unit based on an optical coherence tomography image, or corresponding image data, taken from the reconstruction pattern as applied to the surface of the specimen.

Since the histological tissue slices are generated from the histological specimen by a decomposition of the surface to which the reconstruction pattern has been applied along slice contours intersecting the reconstruction pattern, the two-dimensional images for which the slice image data providing unit is configured to provide image data can be expected to show edge marks on respective edges of the tissue included in the histological slices. Based on these edge marks, particularly their distances with respect to each other in the two-dimensional images corresponding to the histological slices, and further based on the data indicative of the applied reconstruction pattern as provided by the reconstruction pattern providing unit, the reconstruction unit can infer a position of the histological tissue slices in three-dimensional space and hence recompose the slices such that image data for a volumetric model of the histological specimen are obtained. In orderto detect the edge marks and associate them to the reconstruction pattern, or reconstruction pattern parts, from which they originate, which is preferably an automatic process, the reconstruction unit may be configured to take into account a width, a depth and a multiplicity or other distinguishing features of the edge marks. In particular, the reconstruction unit may be configured to use a trained artificial neural network, more particularly cascade neural networks, for this purpose.

The result of the three-dimensional histopathological reconstruction, i.e. the volumetric image of the histological specimen has manifold applications. For instance, it may be used for training artificial intelligence-based endoscopic, laparoscopic or intraoperative systems. Such systems have so far been trained through a labelling of endoscopic images by experts, or by "global" histopathological validation. Training the systems using the gold standard of "local" histopathological diagnostics may have particular relevance for findings with complex configurations such as, for instance, carcinoma on the bottom of a Barrett oesophagus, or for detecting polyps endoscopically. Apart from training artificial intelligence-based endoscopic systems, the generated volumetric images may also be used for establishing neuroanatomical and functional atlases of brain tissue or other tissue structures having an unstable structure and smooth surfaces. Generally, the generated volumetric images allow for richer information on three-dimensional tumour morphologies, a vicinity of tumours to resection margins or other cutting surfaces as well as a positioning of specimens with respect to a patient anatomy, all of which can improve, particularly in combination, medical care, particularly regarding tumour diagnoses. Direct applicability is expected for diagnoses regarding prostate specimens, for which the importance of positioning the specimen with respect to the surrounding prostate tissue has been considered to be particularly high. Also, applications to particularly large specimens are envisaged, which need to be partitioned into individual specimen parts. Specifically, an application to mammal lumpectomies seems to be of interest. Besides, as already indicated further above, applications of the techniques disclosed herein to hollow organs such as the oesophagus or the colon are proposed, since a histological three-dimensional reconstruction of tissue specimens of this type using known techniques based on fiducial markings is considered impossible. In addition, the techniques disclosed herein may be used for verifying other imaging techniques based on the histological gold standard. For instance, hyperspectral imaging techniques as applied in endoscopy may be verified in this manner.

As discussed further above regarding the background of the invention, the surgical extraction of tissue, its (optional) partitioning into individual histopathological specimens, and the slicing of the one or more specimens results in a loss of spatial information. A three-dimensional histological reconstruction of a specimen from its two-dimensional histological slices, as described above, allows to recover a spatial relation of the histological slices with respect to each other, but not a spatial relation of the specimen with respect to an anatomical region from which it stems. In particular, information regarding an orientation of an extracted tissue part with respect to anatomic structures and body axes can be lost by the extraction. Conventionally, orientation of the original specimen with respect to anatomic landmarks and body axes typically relies on the surgeon who has extracted the specimen and the pathologist carrying out the histological workup, and additionally on fiducial markings such as, for instance, needles, ink or coagulation markings, applied during or directly after surgery. However, there is a need for less invasive and/or more accurate means for providing information on a spatial relation between an extracted tissue specimen with respect to its anatomic origin. This need is present independently of any reconstruction carried out and, in fact, has caused major concerns: If pathology diagnoses an insufficient distance between a tumour and a resection margin, failure of retrospective specimen orientation can lead to wrong, unnecessarily large (and traumatizing) or insufficiently small re-operations.

The above-mentioned need is met by an apparatus for marking specimen tissue to be extracted from an anatomical region in a subject's body, wherein the apparatus comprises a marking device for marking the specimen tissue inside the anatomical region, wherein the marking device comprises a pulsed infrared laser for generating pulsed infrared light, and a light guide for guiding the laser light from the infrared laser towards the anatomical region in the subject's body. Using this apparatus, the surgeon extracting the specimen can apply markings to the specimen *in situ,* i.e. while the specimen has not yet been extracted from its anatomical surroundings. The risk that a wrong marking, such as a marking referring to an incorrect orientation of the extracted specimen, is applied can thereby be decreased. Moreover, while the laser is not necessarily a pulsed infrared laser, but could also be a different laser, by using a pulsed infrared laser light for applying the markings cold vaporisation can be effected, thereby allowing for virtually invisible markings causing no significant tissue damage. The laser can particularly be a micro-, picosecond or nanosecond infrared laser.

The marking device can be a hand-held device or a device fixed to a hand-held surgical instrument. For instance, the light guide can lead through a working channel of a surgical instrument and/or along an endoscope to a tip thereof. Hence, the marking device can be configured to allow a user, such as the surgeon, to manually apply arbitrary markings at his/her discretion. For instance, the user can use the marking device to encircle a region to which the pathologist should pay particular attention. Additionally or alternatively, the marking device may be configured to allow for an automatic or semiautomatic application of specific markings. Regular markings, such as a set of straight lines, for instance, may be applied using one or more mirrors at a distal end of the light guide, wherein the mirrors may be movable such that a light beam exiting the light guide onto the mirrors and reflected by the mirrors is directable onto a surface of the tissue region to be extracted, i.e., for instance, along the marking's straight lines. An automatic or semiautomatic marking like this may involve a control by a computer.

The markings applied to the specimen using the marking device, i.e. the *in situ* markings, may later be mapped to a volumetric reconstructed model of the specimen. For instance, the markings may be included in the volumetric image for which the reconstruction unit reconstructs the three-dimensional image data. This could mean, in particular, that, in the volumetric image, a natural specimen surface appears with the markings applied to it *in situ.* The markings can also be included in the two-dimensional images of the individual histological slices, in order to visualise tissue regions marked by a surgeon also to the pathologist. Also, the markings can be included in the image data acquired for verifying whether a reconstruction pattern has been applied correctly, particularly in the optical coherence tomography image data, in order to, for instance, orient the specimen also in these images.

The apparatus comprising the marking device may include a camera for filming the *in situ* application of the markings, i.e. for acquiring *in situ* images. For example, the camera can be a camera which is already present in or at an instrument and/or endoscope, or it can be an additional camera. If, for instance, the marking device comprises a surgical instrument and/or endoscope through or along which the light guide leads, the camera may be positioned at a distal end of the instrument and/or endoscope, respectively. The *in situ* markings may then not only be identified in the film, but also in one or more images acquired with the imaging device used for verifying the application of the reconstruction pattern (e.g., one or more OCT images) and one or more histological slice images. This allows to determine a position, particularly an orientation, of individual histological slices and corresponding slice images with respect to the *in situ* film, and hence with respect to possibly pre- or periinterventionally acquired images.

Additionally or alternatively to markings indicating a spatial relation of the specimen to be extracted with respect to its anatomical surroundings, also a reconstruction pattern as discussed in detail above can be applied using the presently described apparatus. The marking device can therefore be configured to mark the specimen tissue by applying a reconstruction pattern to a surface thereof that is decomposable along slice contours arising from a histological slicing to be carried out on the specimen tissue after extraction, wherein the applied reconstruction pattern intersects the slice contours so as to be decomposed by the histological slicing and restored upon three-dimensional histological reconstruction.

Hence, reconstruction patterns can be applied both *in vivo*/*in situ* as well as ex *vivo.* Moreover, one and the same set of markings/pattern can fulfil both functions, i.e. the function of indicating a spatial relation between the tissue region to be extracted with respect to its anatomical surroundings and the function of indicating a spatial relation between the histological slices to be generated by the histological slicing of the extracted specimen for facilitating a three-dimensional histological reconstruction. For instance, the marking device, which may insofar also be regarded as a reconstruction pattern application device, may be configured to apply the reconstruction pattern to a tissue region in the subject's body from which the histological specimen is to be extracted such that the extraction of the specimen from the tissue region results in a remaining part of the reconstruction pattern remaining in the tissue region and an extracted part of the reconstruction pattern formed on a surface of an extracted part of the tissue region corresponding to the histological specimen. The surface of the extracted part of the tissue region may be the surface that will be decomposed along slice contours arising from the planned, predefined histological slicing.

In a further aspect, a display device is presented which is configured to display markings applied to specimen tissue to be extracted from an anatomical region in a subject's body in at least one of a) a two-dimensional image corresponding to a histological slice generated by a histological slicing carried out on the specimen tissue after being extracted, b) an image of an applied reconstruction pattern on the specimen tissue, and c) a volumetric image of the specimen tissue generated based on a three-dimensional histological reconstruction. A surgeon and a pathologist can use such a display as a "common ground" to exchange information about medical findings, particularly in a manner allowing for a positioning of the findings with respect to both a patient anatomy and the specimen as a whole. Since the markings applied to the specimen tissue before extraction, i.e. *in situ,* translate to the extracted specimen and therefore also to the histological slices generated based on the extracted specimen, wherein the three-dimensional reconstruction is carried out based on the histological slices, already the three-dimensional reconstruction by itself may lead to the markings applied to the specimen tissue before extraction being visible in a volumetric image of the reconstructed histological specimen. A display of the markings in an image of the applied reconstruction pattern of the specimen tissue, such as the optical coherence tomography images, for instance, may be achieved by a registration of such an image with the reconstructed volumetric image, and/or by a registration of such an image with an image acquired of the specimen tissue *in situ.*

When filming the *in situ* application of markings as described above and additionally filming the specimen before or during application of the reconstruction pattern, i.e. after extraction, via registration of the film material and the histological reconstruction, a "seamless image chain" can be achieved. Image data before *in situ* marking, image data including the *in situ* markings, image data including both *in situ* markings as well as the reconstruction pattern applied after specimen extraction, particularly in the form of OCT image data, and reconstructed volumetric image data can be related to each other and used for display on the display apparatus. For instance, images of the reconstructed three-dimensional model may be displayed which include the markings applied *in situ,* the reconstruction pattern applied after specimen extraction, and an anatomical neighbourhood from which the specimen has been extracted (e.g., surrounding tissue structures as filmed *in situ*)*,* including the possibility of viewing the image data along two-dimensional sections, such as, for instance, sections corresponding to the histological slice images acquired along the way, based on which the reconstruction has been carried out.

It is understood that the present disclosure also relates to methods and uses corresponding to the above described apparatuses and systems.

Accordingly a method for facilitating three-dimensional histological reconstruction is presented, wherein the method includes applying a reconstruction pattern to a surface of a histological specimen, wherein the surface will be decomposed along slice contours arising from a predefined histological slicing to be carried out on the specimen, wherein the applied reconstruction pattern intersects the slice contours so as to be decomposed by the histological slicing and restored upon three-dimensional histological reconstruction. The method can be carried out by using the apparatus for facilitating three-dimensional histological reconstruction.

Furthermore, a method for three-dimensional histological reconstruction is presented, wherein the method includes data indicative of a reconstruction pattern that has been applied to a surface of a histological specimen, providing image data for two-dimensional images of histological tissue slices generated from the histological specimen by a decomposition of the surface to which the reconstruction pattern has been applied along slice contours intersecting the reconstruction pattern, and reconstructing three-dimensional image data for a volumetric image of the histological specimen based on the data indicative of the applied reconstruction pattern and the image data for the two-dimensional images of the histological slices such that, in the volumetric image, the reconstruction pattern is restored on the surface of the histological specimen. The method can be carried out by using the system for three-dimensional histological reconstruction.

Also, a method for marking specimen tissue to be extracted from an anatomical region in a subject's body is presented, wherein the method includes marking the specimen tissue inside the anatomical region by generating pulsed infrared laser light and guiding the laser light to the anatomical region in the subject's body. The method can be carried out by using the apparatus for marking specimen tissue to be extracted from anatomical region in a subject's body.

In a further aspect, a computer programme for facilitating three-dimensional histological reconstruction is provided, wherein the program comprises instructions causing the apparatus for facilitating three-dimensional histological reconstruction to carry out the method for facilitating three-dimensional histological reconstruction if the programme is run on a computer controlling the apparatus.

Moreover, a computer programme for three-dimensional histological reconstruction is presented, wherein the program comprises instructions causing the system for three-dimensional histological reconstruction to carry out the method for three-dimensional histological reconstruction.

Additionally, a computer programme for marking specimen tissue to be extracted from an anatomical region in a subject's body is presented, wherein the program comprises instructions causing the apparatus for marking specimen tissue to be extracted from an anatomical region in a subject's body to carry out the method for marking specimen tissue to be extracted from an anatomical region in a subject's body if the programme is run on a computer controlling the apparatus.

It shall be understood that the apparatus of claim 1, the system of claim 10, the apparatus of claim 11, the display device of claim 13, the method of claim 14 and the computer program of claim 15, as well the subject-matter of the related aspects described above or below, have similar and/or identical preferred embodiments, in particular, as defined in the dependent claims. It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
- Fig. 1: shows schematically and exemplarily an apparatus for facilitating three-dimensional reconstruction,
- Fig. 2: shows schematically and exemplarily a view onto a surface of a specimen on which a reconstruction pattern has been applied,
- Fig. 3: shows schematically and exemplarily a set of histological slice images,
- Fig. 4: shows schematically and exemplarily non-flat specimens with surfaces on which reconstruction patterns have been applied,
- Fig. 5: shows an exemplary photographic image of a specimen surface on which a reconstruction pattern has been applied,
- Fig. 6: shows an exemplary optical coherence tomography image of the specimen surface shown in Fig. 5,
- Fig. 7: shows schematically and exemplarily an apparatus for marking specimen tissue to be extracted from an anatomical region in a subject's body,
- Fig. 8: shows schematically and exemplarily a system forthree-dimensional histological reconstruction, and
- Fig. 9: shows schematically and exemplarily a method for three-dimensional histological reconstruction.

### DETAILED DESCRIPTION OF EMBODIMENTS

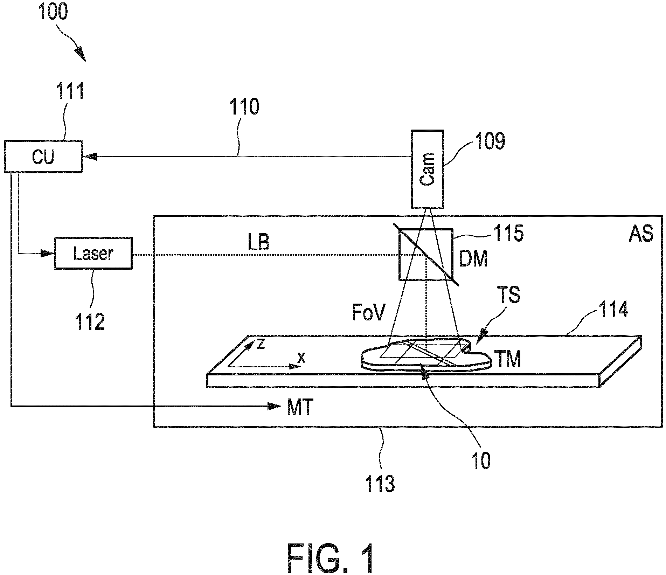

Fig. 1 shows schematically and exemplarily an apparatus 100 for facilitating a three-dimensional histological reconstruction. The apparatus comprises a camera ("Cam") 109 whose field of view ("FoV") is directed onto a histological tissue specimen ("TS") that is placed on a mechanical table ("MT") movable along two perpendicular horizontal directions, in this case the x-direction and the z-direction. The apparatus 100 further comprises an air sealing ("AS") 113 encapsulating the mechanical table, in order to allow for a processing of fresh human tissue specimens. The camera acquires images of the specimen through the air sealing and provides the acquired images via an image providing unit 110 to a control unit ("CU") 111.

In the exemplary embodiment schematically shown in Fig. 1, the histological tissue specimen is an endoscopically retrieved specimen from the gastrointestinal tract. As seen in Fig. 1, such specimens are characterised by a relatively large extension in the directions of the original organ surface and a comparatively smaller extension in the direction perpendicular to the original organ surface. Accordingly, the specimen in Fig. 1, as placed on the mechanical table, appears flat, wherein the x-z-plane corresponds to the directions parallel to the original organ surface.

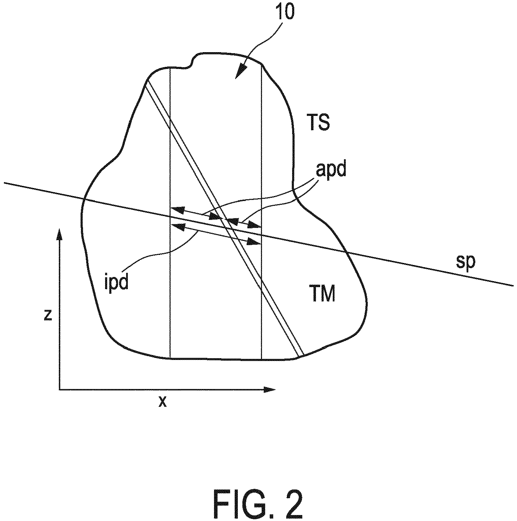

In the illustrated embodiment, a histological slicing is predefined to be carried out on the specimen in a direction lying in the x-z-plane, as will be further detailed with respect to Fig. 2. Typically, the sectioning planes for the histological slicing would be x-y-planes, for instance. Hence, the diagnostic focus lies on tissue structures visible in the long and flat, "strip-like" slices arising from a sectioning of the flat tissue specimen lying on the mechanical table with planes perpendicular to the table plane, i.e. with planes comprising the y-direction. Due to the predefined histological slicing, the top surface of the tissue specimen visible in Fig. 1 will be decomposed along slice contours. Since, in the illustrated embodiment, the tissue specimen is flat, the slice contours will correspond to flat lines perpendicular to the slicing direction.

The top surface of the tissue specimen, i.e. the surface of the specimen 10 that will be decomposed by the histological slicing along the slice contours corresponding in this case to straight lines, is viewed by the camera. Hence, the image data providing unit 109 is configured to provide image data for two-dimensional images of the histological specimen that correspond to a view onto the surface of the specimen that will be decomposed by the histological slicing. As the mechanical table moves along the x- and z-direction, the specimen can appear at different positions in the field of view and therefore in the two-dimensional images at different times. For instance, the acquired images may be rasterised images.

The control unit 111 of the apparatus comprises, or acts as, a reconstruction pattern determining unit configured to determine a reconstruction pattern ("TM") to be applied to the surface of the specimen viewed in the camera's images based on the predefined histological slicing and the image data received via the image data providing unit 109. The reconstruction pattern to be applied to the specimen surface, which can be understood as a particular kind of tissue marking, is determined such that it intersects the slice contours that will arise from the predefined histological slicing so as to be decomposed by the histological slicing and restored upon three-dimensional histological reconstruction. Moreover, the reconstruction pattern determining unit is configured to determine a boundary of the surface of the specimen in the two-dimensional images acquired by the camera based on the corresponding image data received via the image data providing unit, and to determine the reconstruction pattern based on the determined boundary. For this purpose, the reconstruction pattern determining unit may be configured to apply a thresholding, morphological operations, particularly morphological opening and closing, machine learning models, or a combination of these techniques. For instance, the reconstruction pattern determining unit is configured to select, out of a predefined set of reconstruction line patterns, an optimal one for the shape of the specimen given by the determined boundaries and, if necessary, scale the chosen reconstruction line pattern such that the entire specimen surface is covered. The choice of reconstruction line pattern may be made dependent on the slicing direction of the predefined histological slicing.

The apparatus 100 further comprises a pulsed infrared laser 112 configured to emit a laser beam ("LB") into the air sealing and onto a dichroitic mirror ("DM") 115 directing the laser beam onto the surface of the specimen imaged by the camera. While the laser light is already restricted in wavelengths to the infrared range, the dichroitic mirror helps to further narrow down the spectrum so as to keep the thermal impact on the tissue specimen as minimal as possible while still allowing for an application of the reconstruction pattern. The reconstruction pattern, which is in this case a reconstruction line pattern, is applied to the surface of the specimen viewed by the camera by operating the laser 112 and the mechanical table 114 accordingly. The laser 112 and the mechanical table 114 may be operated by the control unit. Additionally or alternatively to moving the mechanical table, also the laser 112 and/or the dichroitic mirror 115 may be movable, possibly also by control via the control unit 111, so as to apply the reconstruction pattern to a surface of the specimen. Either while the reconstruction pattern is applied or once the application of the reconstruction pattern has been finished, one or more images of the specimen surface may again be acquired using the camera and/or a further imaging device, such as an optical coherence tomography device. The images acquired after applying the reconstruction pattern can be used by the control unit 111 to very if, or to which degree, the reconstruction pattern has been accurately applied. This can include determining the reconstruction pattern as applied and comparing it to the originally intended reconstruction pattern. The reconstruction pattern as applied can also be provided as a basis for the subsequent reconstruction, as will be described further below.

Since a laser is used to apply the reconstruction pattern, mechanical stress on the specimen can be avoided. This may generally be preferred, but will be so particularly for low stability specimens such as mucosal tissue specimens. As exemplified by specimens from the gastrointestinal tract, which comprise mucosal tissue and additionally parts of the submucosal layer, existing three-dimensional reconstruction techniques have been found to be practically not applicable to such specimens, particularly if they are flexible before and even after formalin fixation. In routine histopathological processing, the specimens are typically cut into different blocks before paraffin embedding or even fixation, due to their large extension in one of the x- and the z-direction. The apparatus 100 allows for a three-dimensional reconstruction of endoscopic specimens and thereby overcomes the limitations in the prior art. It may be used as an alternative or in combination with known three-dimensional reconstruction techniques for other specimens, i.e. specimens which are amendable to the known techniques, such as prostate cancer specimens or brain, particularly mice brain, specimens.

Fig. 2 shows schematically and exemplarily the tissue specimen from Fig. 1 as it would appear in an image acquired by the camera of the apparatus 100. In Fig. 2, one of the planned sectioning planes ("sp") for the histological slicing to be carried out on the specimen is indicated by a line. The line could be understood as indicating the line of intersection between the sectioning plane, which would preferably be perpendicular to the x-z-plane, and the surface of the specimen to which the reconstruction pattern is to be applied, which is in this case substantially flat.

Apart from the sectioning plane, the reconstruction pattern already indicated in Fig. 1 is shown again in Fig. 2. In the illustrated example, the reconstruction pattern consists of two parallel lines in z-direction and a third line crossing the two parallel lines. The third line is provided with a multiplicity of two, thereby providing a feature distinguishing it from the two parallel lines. As seen in Fig. 2, the multiplicity of two of the third line refers to the circumstance that the third line actually consists of two parallel sublines, wherein a distance between the two parallel sublines forming the third line of the reconstruction pattern is significantly smallerthan a distance between the first two parallel lines (main lines) of the reconstruction pattern. Already this difference in line distance allows to distinguish the sublines forming the third line from the first two parallel lines. However, the sublines may additionally be provided with distinguishing features like a different width or a different depth as compared to the first two lines in the reconstruction pattern. All these distinguishing features can be achieved by repeated laser ablations, possibly with different beam foci and/or intensities.

The reconstruction line pattern exemplarily illustrated in Fig. 2 is chosen such that, like the sectioning plane exemplarily indicated in Fig. 2, every sectioning plane of the histological slicing to be carried out on the specimen, more particularly every of the contour lines along which the respective sectioning planes intersect the specimen surface, will intersect each of the lines in the reconstruction line pattern exactly once. The intersections between the lines of the reconstruction line pattern and the contour lines arising from the histological slicing on the specimen surface correspond to edge marks that will appear on the edges of the histological slices. In the two-dimensional images of the histological slices, distances between the edge marks can be measured that correspond to the distances between the intersections of contour lines and reconstruction lines indicated in Fig. 2. Specifically, for the exemplary reconstruction line pattern of Fig. 2, an inter-parallel line distance ("ipd") can be identified that corresponds to a distance between the edge marks arising from the first two parallel lines of the reconstruction line pattern, and angulated-to-parallel-line distances ("apd") that correspond to the distances of the edge marks of a respective one of the first two parallel lines in the reconstruction pattern to the edge mark arising from the third, angulated line in the reconstruction pattern. The orientation of a sectioning plane of the histological slicing, and hence of the corresponding histological slice, can then be retrieved as follows: First, the inter-parallel line distance is extracted to compute the angle against the x-axis. Second, the angulated-to-parallel line distances are extracted to obtain the location, or offset, along the z-axis. The location along the x-axis can be measured directly from the intersection points with, for instance and preferably, the parallel lines.

The location along x can additionally be computed from the angle against the x-axis, the location along the z-axis and the boundary of the specimen, under the assumption that the individual tissue section, or histological slice, extends to at least one of the resection boundaries (which may be violated in cases where individual tissue sections are damaged in the course of the histopathology workup). This provides an additional means to measure the position along x, hence to estimate the reconstruction precision.

It should be noted that the *relative* position of any two such sections, or slices, in x naturally follows from their intersection points with the parallel lines, as it is known that these intersections stem from two straight, parallel lines. A number of, for instance, parallel lines hence allow for a robust measurement for the relative position of any two tissue sections in x, also in the case of serious tissue artefacts (missing parts at the resection boundaries, tissue deformations upon processing/paraffin embedding etc.). Also in these cases, it is still known that intersection points from a single line need to form a straight line in the reconstructed 3d-model, hence the tissue sections need to be positioned and/or tilted accordingly. In this context, it should be noted that individual lines can be applied such that they are distinguishable from their intersection points alone by, e.g., different widths, depths, and multiplicities.

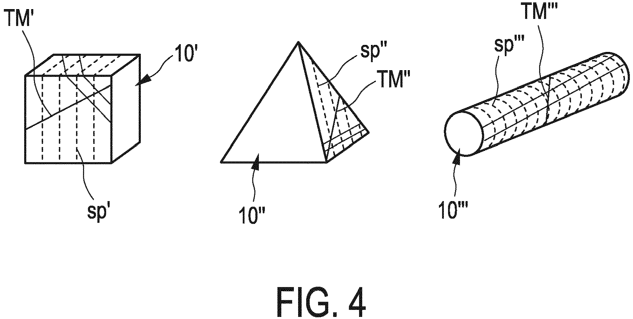

The remaining parameters to a full positioning in the six-dimensional configuration space - namely, angle against the y- and z-axis, location along y - can, in the case of a flat specimen, be neglected (or, in the case of location along the y-axis, be retrieved from anatomical assumptions, e.g. assuming one or more basal lamina sheets of the specimen to be flat). For non-flat specimens, the remaining parameters can be computed from an application of analogous patterns on other specimen surfaces. In other words, for non-flat specimens, the positioning of the histological slices can be carried out in different spatial dimensions/on different surfaces of the specimen. To illustrate this aspect, as will be appreciated from Fig. 2, the line of intersection between the indicated sectioning plane and the flat specimen surface is independent of a rotation of the sectioning plane about the intersecting line. Hence, an orientation of the corresponding histological slice can only be determined up to an angle corresponding to this rotation. If the specimen had a further surface which the sectioning plane intersected, also a reconstruction pattern on that further surface would have to be applied in order to fully determine the orientation of the sectioning plane and hence the position of the histological slice in the specimen volume. This aspect will be further elucidated with reference to Fig. 4.

It should be noted that, if furthermore assuming parallelism and equidistance of the tissue sections, or slices, a single straight line would suffice to deduce the position along x, hence complete the positioning in the six-dimensional configuration space. In this sense, already a reconstruction pattern consisting of a single line may be sufficient, wherein adding a further, parallel line, may allow for dropping a first assumption regarding a respective angle of the tissue sections against the x-axis can be inferred, whereas additionally or alternatively adding a further, angulated, or non-parallel, line may allow for dropping a second assumption regarding a respective location of the tissue sections in z-direction. Hence, depending on the number of assumptions made, a single line, two parallel or non-parallel, or two parallel and one non-parallel, angulated line may be chosen for building up the reconstruction pattern. It will of course be understood that these are just four examples, and many other reconstruction patterns are possible, wherein other reconstruction patterns may, for instance involving more lines or also other constituents than lines.

It should further be noted that any pattern with redundant information beyond what is needed for deriving the position of individual tissue sections can be used to also correct for deformations within individual tissue sections, e.g. (irregular) shrinkage or deflection of the tissue which may occur after tissue extraction and during the histopathology workup. For instance, a family of straight parallel lines with defined inter-parallel distances can be used to correct for (heterogeneous) tissue shrinkage.

It should be understood that the process of tissue marking and imaging, preferably by OCT, can partly or entirely be undertaken before or after specimen fixation by formalin, or both e.g., by applying one pattern before fixation, imaging of it before and after fixation; possibly, another pattern can be applied after fixation.

Particularly, by application of a pattern before fixation and imaging, preferably through OCT, before *and* after fixation, through comparison of the two (OCT-) imaged patterns, shrinkage and deformations of the specimen due to formalin fixation can be quantified and corrected for.

Furthermore, it may not always be possible in practice to apply the reconstruction pattern to a specimen surface with perfect precision. Moreover, also the distance measurement between the edge marks appearing in the histological slices may not be perfect in reality. Even in case of a flat specimen surface, in which case the distances between the edge marks correspond to the distances in the reconstruction pattern as visible in a top view like in Fig. 2, the distance measurements may only be possible with a limited precision. The position of the histological slices can be determined with an increased accuracy by repeating the distance measurements with respect to further reconstruction patterns. The further reconstruction patterns can be the same as the first reconstruction pattern (preferably up to distinguishing features distinguishing its elements, such as its lines, from the elements of the first reconstruction pattern) at different locations and/or with different orientations than the first reconstruction pattern, and/or may be different reconstruction patterns.

Hence, applying more than one reconstruction pattern to the specimen may not only be preferred for determining a full position of the histological slices in six-dimensional configuration space, which may particularly be necessary for non-flat specimens, but also for increasing a precision of the positioning, due to an added redundancy.

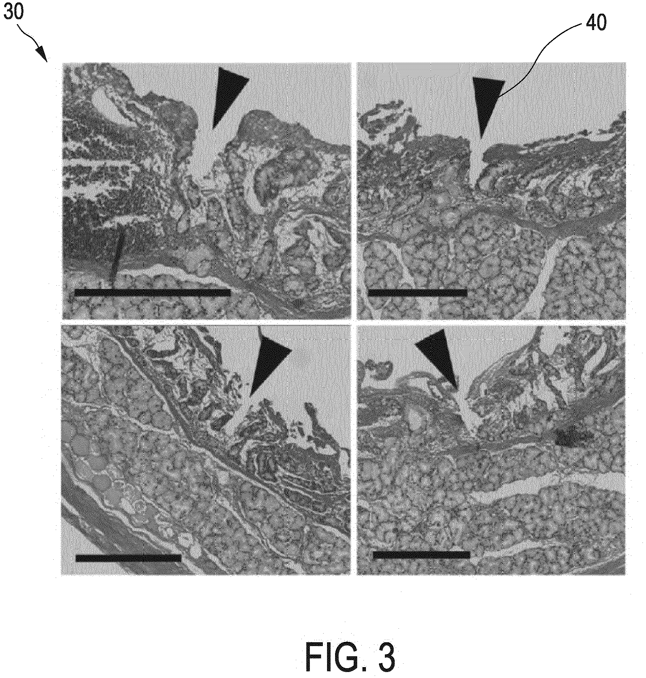

Fig. 3 shows four exemplary images 30 corresponding to histological slices taken from a tissue specimen like the one shown in Fig. 1 and Fig. 2, in this case taken from porcine duodenum. The tissue has been stained, as is characteristic for a histopathological workup, in this case using haematoxylin and eosin (HE stain). The scale bars in the lower left corners of the images correspond to 100 µm. The triangular annotations added to the images point at edge marks 40 corresponding to the intersections of a previously applied reconstruction line pattern with the contour lines along which the specimen surface has been decomposed during histological slicing. The reconstruction line pattern has in this case been applied using a nanosecond pulsed infrared laser (NIRL) to effect cold vaporisation, wherein lines that have caused the edge marks 40 seen in the image of Fig. 3 have been applied substantially perpendicular to the sectioning planes of the histological slices. As will be appreciated from Fig. 3, the damage caused to the tissue remaining after the laser ablations is relatively low. The indentations into the tissue structure corresponding to the edge marks 40 appear relatively narrow and distinct, yet are clearly visible in the images 30, thereby allowing for an accurate distance measurement between the edge marks within the histological slices and not negatively affecting the histopathological analysis.

Fig. 4 shows schematically and exemplarily three possible alternative geometries for histopathological specimens to which reconstruction patterns could be applied for facilitating a three-dimensional histological reconstruction following a histological slicing. In the latter case, a specimen 10' having a cubic shape is shown, wherein a histological slicing is predefined to be carried out along sectioning planes sp'. As a reconstruction pattern, a line pattern TM' similarly to the line pattern illustrated in Fig. 1 and Fig. 2 is shown, but in this case the single line pattern TM' becomes three-dimensional, since it is extended over different sides, or surfaces, of the cubic specimen 10'. If this were not the case, i.e. if the line pattern were restricted, for instance, to the front side of the specimen 10', then it would not suffice for a full three-dimensional histological reconstruction, since the edge marks visible on the histological slices would carry insufficient spatial information. It should be noted that the reconstruction TM' is only sufficient due to the particular orientation of the sectioning planes sp'. If the slicing direction were, for instance, rotated clockwise by 90 degrees in the figure, such that contour lines would be generated on the side surface of the cube, the reconstruction pattern would need to be extended, or additional reconstruction patterns would need to be added, to the side surfaces.

In the middle of Fig. 4, a specimen 10" having a pyramidal shape is schematically and exemplarily shown. In this case, the reconstruction line pattern TM" is insufficient for a full three-dimensional reconstruction of the histological slices along the sectioning planes sp", since its lines intersect with the contour lines of the histological slices only on the one side of the specimen 10", the side appearing as a right side in Fig. 4. Therefore, the reconstruction pattern TM" could be extended, for instance, to the surface of the specimen appearing as a front surface in Fig. 4 - even though this might amount in this case to learning, from a lack of intersections of contour lines with the extended line pattern on the front surface, that the sectioning planes sp' lie actually parallel to the front surface.

On the right in Fig. 4, a specimen 10" having a cylindrical shape is schematically and exemplarily shown. In the illustrated example, the histological slicing is predefined to be carried out along the axis of symmetry of the cylinder as a slicing direction, resulting in sectioning planes sp‴. A reconstruction line pattern TM‴ is applied to a lateral surface of the cylinder-shaped specimen 10‴. As long as the extrinsic curvature of the lateral surface is properly taken into account when inferring the inter-parallel line distance between the parallel lines of the reconstruction line pattern TM'" from the histological slices, a single reconstruction pattern can in this case, in principle, be sufficient for the three-dimensional histological reconstruction.

Particularly for non-flat specimens like those schematically and exemplarily shown in Fig. 4, it may be preferred that the image data providing unit is configured to provide image data for a plurality of two-dimensional images of the respective histological specimen, wherein each of the images corresponds to a different view onto the surface of the specimen, or in fact a view onto a different surface of the specimen, wherein the reconstruction pattern determining unit is configured to determine the reconstruction pattern with respect to each of the images - or, in fact, to determine a plurality of reconstruction patterns with respect to the plurality of images. Hence, the camera of the apparatus 100 may be configured to acquire images with different fields of view, such as from different positions and/or with different viewing angles.

It should be emphasised that, although non-flat specimen shapes like those schematically and exemplarily illustrated in Fig. 4 may increase the complexity, a three-dimensional histological reconstruction is still possible, as long as one or more appropriate reconstruction patterns are applied.

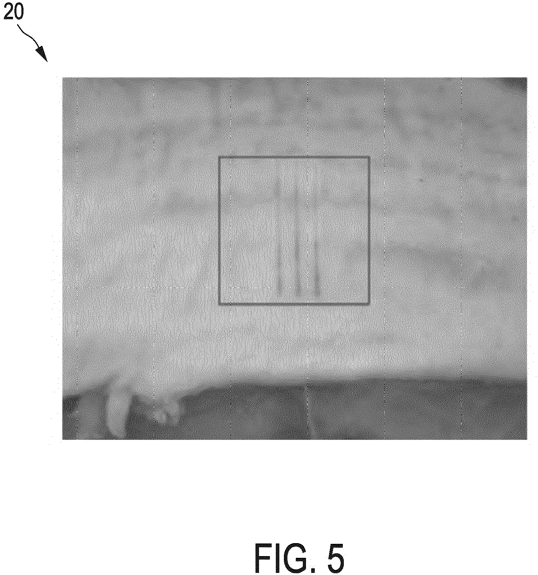

Fig. 5 corresponds to a photographic image 20 of a surface of a tissue specimen onto which three parallel lines, which could form a reconstruction pattern or a part of it, have been applied. Images like the one shown in Fig. 5 could also be acquired by the camera of the apparatus 100 before the reconstruction pattern has been applied, in order to determine, particularly select and/or scale, the reconstruction pattern to be applied based thereon, such as based on the specimen boundaries detected therein, for instance. The photographed specimen is in this case porcine gastric mucosa, i.e. tissue from the inner wall of a pig's stomach. The shown parallel lines have been applied, using a nanosecond infrared laser, at an inter-parallel line distance of 500 µm and with a line width of about 130 µm. The line width could also be decreased. For instance, alternatively a line width of 40 µm could be achieved.

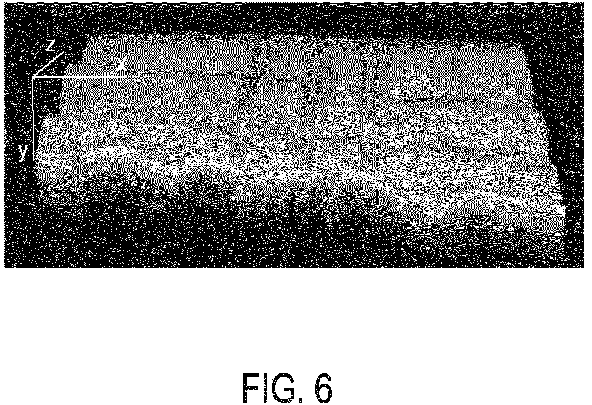

Fig. 6 corresponds to an optical coherence tomography image of the specimen shown in Fig. 5. In this image, the inter-parallel line distance between the three applied lines can be measured more accurately than in the photographic image of Fig. 5. Therefore, particularly optical coherence tomography images can be used for verifying the application of reconstruction patterns to a specimen surface. Hence, the apparatus 100 can comprise, particularly in addition to the camera already shown in Fig. 1, an imaging device such as an optical coherence tomography imaging device for acquiring verification images of the reconstruction pattern as applied to the specimen. The control unit 111 of the apparatus 100 may be configured to control a further application of the reconstruction pattern, i.e. the movement of the laser, the mechanical table and/or the mirror, based on the verification images. Alternatively, the subsequent histological reconstruction can be made based on corrected positioning data inferred from distance measurements between edge marks as described above, wherein the correction may be achieved based on the reconstruction pattern as appearing in the verification images, as opposed to the reconstruction pattern as originally intended.

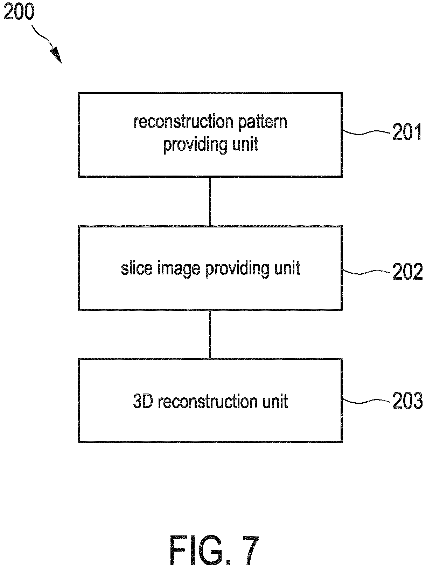

Fig. 7 shows schematically and exemplarily a system 200 for three-dimensional histological reconstruction. The system comprises a reconstruction pattern providing unit 201 configured to provide data indicative of a reconstruction pattern that has been applied to a surface of a histological specimen, and a slice image data providing unit 202 configured to provide image data for two-dimensional images 30 of histological tissue slices generated from the histological specimen 10 by a decomposition of the surface to which the reconstruction pattern has been applied along slice contours intersecting the reconstruction pattern. The system 200 further comprises a reconstruction unit 203 configured to reconstruct three-dimensional image data for a volumetric image of the histological specimen based on the data indicative of the applied reconstruction pattern and the image data for the two-dimensional images of the histological slices such that, in the volumetric image, the reconstruction pattern is restored on the surface of the histological specimen. Hence, the three-dimensional histological reconstruction of the specimen, which can be facilitated by the apparatus 100 through the application of the reconstruction pattern, can be subsequently be carried out by the system 200.

As described further above, an appropriately chosen and applied reconstruction pattern allows for full three-dimensional positioning of the individual histological slices via a detection of the edge marks 40. Knowing the three-dimensional position, i.e. the three-dimensional location of the histological slices and their spatial orientation, allows to recompose a model of the specimen based on the histological slices, thereby generating a volumetric histological model of the specimen. Hence, the reconstruction unit may be configured to detect the edge marks 40 in the histological slice images 30, determine distances between them, and determine the three-dimensional positions of the histological slices based on the determined distances between the edge marks 40 in the histological slice images 30 and the corresponding distances in the applied reconstruction pattern, such as by using known trigonometric formulae. In this case, the reconstruction unit 203 may essentially be an image processing and computation unit. In principle, the three-dimensional histological reconstruction could also be achieved without using known analytic relationships. For instance, the reconstruction unit 203 could be configured to try out various relative positions between the plurality of histological slices and maintain those relative positions in which the reconstruction pattern reappears on the surface of the specimen, i.e. those relative positions in which the edge marks 40 recompose to the reconstruction pattern. Also a combination of the two approaches is possible. For instance, a rough three-dimensional position, or an almost complete three-dimensional position, of the histological slices may be determined by the reconstruction unit 203 based on an analytical prescription, wherein a fine-tuning, i.e. a remaining positioning in three-dimensional space may be achieved by the reconstruction unit based on a trial-and-error approach.

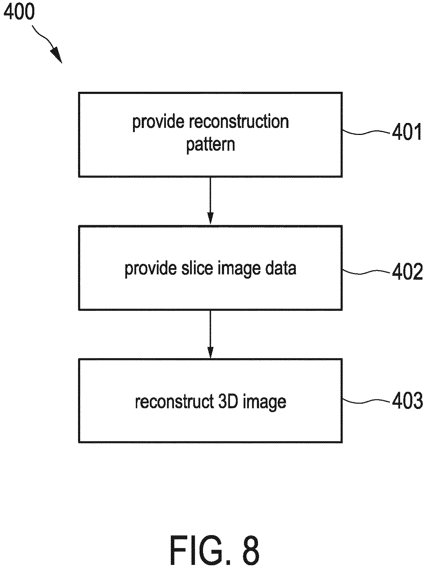

Fig. 8 shows schematically and exemplarily a method 400 that could be carried out by the system 200. The method 400 includes a step 401 of providing data indicative of a reconstruction pattern that has been applied to a surface of a histological specimen, a step 402 of providing image data for two-dimensional images of histological tissue slices generated from the histological specimen by a decomposition of the surface to which the reconstruction pattern has been applied along slice contours intersecting the reconstruction pattern, and a step 403 of reconstructing three-dimensional image data for a volumetric image of the histological specimen based on the data indicative of the applied reconstruction pattern and the image data for the two-dimensional images of the histological slices such that, in the volumetric image, the reconstruction pattern is restored on the surface of the histological specimen.

In a further aspect, it has been realised that, apart from the spatial relationship between the histological slices, which is recovered by virtue of the three-dimensional reconstruction of the specimen from the histological slices, also more accurate knowledge on a spatial relationship of the specimen as a whole with respect to an anatomical region from which it has been extracted would be desirable. While a three-dimensional histological reconstruction, achievable as described above, is able to bridge the gap between the microscopic histopathological analysis and the macroscopic view of the specimen as a whole, this bridge can be extended to further bridge the gap from the macroscopic view of the specimen as a whole to the full patient anatomy. This can help to spatially orient and locate pathological findings with respect to not only a resection specimen itself, but also with respect to the respective entire patient.

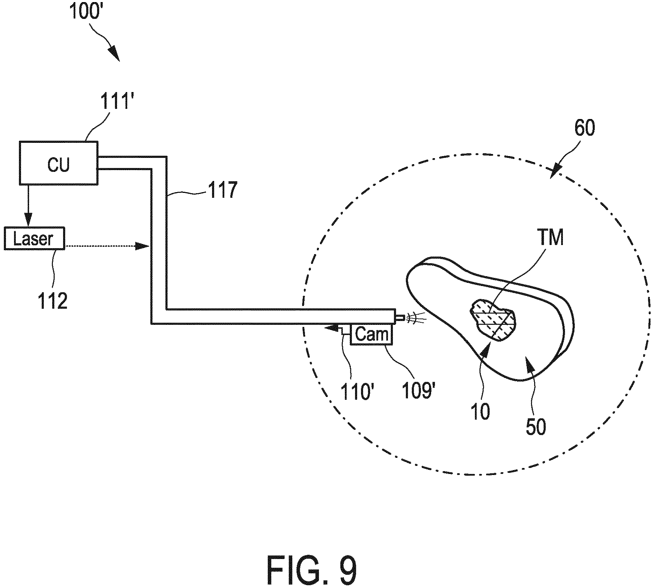

Fig. 9 shows schematically and exemplarily and apparatus 100' for marking specimen tissue to be extracted from an anatomical region 50 in a subject's body 60, wherein the apparatus 100' comprises a marking device for marking the specimen tissue inside the anatomical region 50. The marking device comprises a pulsed infrared laser 112 for generating, for instance, picosecond- or nanosecond-pulsed infrared laser light, and a light guide 117 for guiding the laser light from the laser 112 towards the anatomical region 50 in the subject's body 60. In the example illustrated by Fig. 9, the apparatus 100' further comprises a control unit ("CU") 111' configured to control the laser 112 and possibly also drive means (not shown) so as to drive a motion of the light guide 117 into and inside of the subject's body 60. Furthermore, the apparatus 100' comprises a camera at a distal end of the light guide, wherein the camera is configured to acquire images of the anatomical region 50 when the distal end of the light guide 117 has been placed in a vicinity of the region 50. A corresponding image providing unit 110' is configured to provide the images acquired by the camera to the control unit 111'. In a particular embodiment, the apparatus 100' could correspond to an endoscope into which the light guide 117 has been integrated, wherein the laser 112 is mounted such that its light beam is optically coupled into the light guide 117.

In the example illustrated by Fig. 9, the marking device is configured to apply a reconstruction pattern like the line pattern as discussed above with respect to the apparatus 100, to the specimen tissue to be extracted. Hence, in this aspect it is proposed to apply a reconstruction pattern already before resection of the respective specimen, i.e. while the specimen tissue is still inside the subject's body. As already indicated, the marking device can be part of an endoscope or another medical instrument which a surgeon uses to carry out the resection of the specimen. In this way, a single medical instrument can be used both for marking the specimen tissue before resection and for actually carrying out the resection.

The marking device can comprise mechanics at a distal end of the light guide 117 that are controllable via manual control input means, i.e. a user interface, at a proximal end of the device allowing a user like the surgeon to control how the laser light leaving the light guide at its distal end is directed onto the specimen tissue. In this way, a freehand application of the markings is facilitated. However, since a reconstruction pattern typically will require some regularity and since a user may not be able to provide sufficient accuracy by hand, the apparatus 100' may also comprise, similarly to the mirror 115 of the apparatus 100 if this is movable, movable mirrors at the distal end of the light guide 117, controllable either by hand or automatically by the control unit 111'. Laser light leaving the light guide 117 and being reflected by such movable mirrors can be directed onto the specimen tissue with an improved regularity, thereby facilitating also the application of straight and parallel lines, for instance, to the specimen tissue inside the anatomical region 50.

Instead of marking the specimen tissue to be extracted from the anatomical region 50 by a reconstruction pattern, also different markings may be applied using the apparatus 100'. For instance, simpler markings, which may be sufficient for orienting the specimen tissue with respect to the anatomical region 50, may be applied by hand, wherein the reconstruction pattern may be subsequently applied after resection of the specimen. Such simpler markings may, for instance, include predefined symbols indicating an aboral and an oral side of the tissue to be extracted, respectively, or certain anatomical axes. By OCT imaging, the position of such freehand-markings can be related to the reconstruction pattern. Hence, the position of the freehand-markings can also be determined with respect to the reconstructed 3d-model. This exemplifies again the already discussed possibility of connecting the *in situ* markings with the reconstruction pattern and their joint visualisation in a volumetric image.

It should be emphasised that, while the invention relates to means for facilitating and carrying out a reconstruction of three-dimensional models from histopathological specimen slices, which could be regarded as a first subject (S1), the invention also relates to a periinterventional tissue marking and a determination of a tissue orientation with respect to a patient as a whole, which could be regarded as a second subject (S2).

Regarding the first subject (S1), it has been observed that 3d histological reconstruction traditionally follows one of the following three routes.

Either direct reconstruction from a stack of 2d tissue sections, where, based on the assumption that the shape of a biological specimen changes smoothly, neighbouring tissue sections are registered with respect to each other, usually after correction for processing artefacts, and then stacked to give a 3d volume ("neighbouring" can refer to direct neighbours or a number of tissue sections for improved stability). Due to the inter-sectional smoothness assumption, this requires a sufficient density of the 2d sections, resulting in small volumes to be reconstructed or a massive number of 2d sections - or both. As long as a single paraffin block is considered, this limitation can be overcome by use of so-called "blockface images". For this approach, a camera is mounted directly to the microtome such that it can capture a photograph of the current surface of the paraffin block (hence the name) whilst the latter is cut down slice by slice. The slice-to-slice shift in (in the nomenclature of above Fig. 1 and Fig. 2) x and z direction would have to be computed from these images, and the relative angle of the tissue sections would have to be assumed to be 0° (which holds up to the precision of the microtome and its operator). Blockface techniques are intrinsically restricted to single-block specimens.

Or, second, external ("fiducial") marks are applied to either the specimen itself or the paraffin block and then detected in the 2d sections and used for registration and/or artefact correction. The approach disclosed herein goes along this route. In previous attempts, however, needles have been used to punch holes into the specimen or the paraffin block. The latter - i.e. punching holes into the paraffin block - is again restricted to cases where the entire specimen is processed as a single paraffin block. The former approach, punching the specimen itself, easily introduces serious damage to the tissue, which also means that is can hardly be automated as damage to any region of interest needs to be safely avoided. More importantly, it is restricted to tissue samples that are mechanically stable enough to be punched along the axis which is perpendicular to the later sectioning plane - this does not hold in endoscopically retrieved mucosal tissue.

Alternatively, third, 3d (typically ex vivo) imaging is used as a reference for orienting individual, typically few, tissue sections. This requires either (very) feature-rich specimens - and features that are amenable to both histopathology and the external 3d imaging technique - or again fiducial marks, as in approaches along the second route.

To summarize, each of aforementioned prior techniques is subject to one or a number of the following - intrinsic - limitations: Assumption of slice-to-slice smoothness (direct [slice-to-slice] registration), single-paraffin-block processing (blockface images, paraffin-block needle-punching), mechanically stable specimens (needle-punching of the specimen), availability of a complementary 3d imaging method. None of these requirements is generally fulfilled in endoscopically retrieved tissue specimens.

Regarding the second subject (S2), it has been observed that, to link the perspective of the surgeon, i.e. of the specimen with respect to the patient body, to that of the pathologist, ink or needle markings are usually applied to the specimen directly after resection. Obviously, these can only guarantee a very coarse orientation and, e.g., tell upper from lower end. Precise alignment with respect to an anatomical axis has been found to be impossible. In addition, the "directly after" can be problematic in practice: Interruptions by unexpected events or human error can lead to a wrong application of the ink markings. It is not uncommon that ink markings are wrong by 180°, forgotten, or deliberately omitted due to known loss of orientation.

In addition, surgeons and endoscopists (mis-)use other tools to apply additional markings. This includes application of needles after the resection, with the aforementioned disadvantages plus damage to the tissue. Another (mis-)use with the same intention is the application of additional coagulation spots (by the respective instruments). This is typically performed before the resection, i.e., in situ. This overcomes, to some extent, sources of human error. On the other hand, it damages the tissue that the pathologists shall use for diagnoses. In addition, it may pose a risk to the patient. Precision is typically limited, with the tools being designed for coagulation of bleeding or cutting of tissue, not application of destruction-free markings.

It is proposed herein to use a laser system having the property that tissue remaining after ablation with the laser system is only affected relatively little in order to apply markings to relevant tissue surfaces before and/or after resection, such as, for instance, directly in a tumour and on a mucosal tissue surface. Such a laser system can be used for an automatic and highly precise marking of extracted tissue specimens in order to determine a relative orientation of this two-dimensional histological slices, which amounts to a three-dimensional reconstruction (S1), namely a) in the sense of a non-invasive application of line or other reconstruction patterns directly on the relevant tissue surface, b) which leads to notches, recesses or other edge marks detectable in the individual histological slices at arbitrarily selectable distances and orientations with respect to each other, thereby facilitating a three-dimensional reconstruction, c) by using a laser and a mechanical ("xy-") table to achieve a precise pattern application even on mechanically easily deformable specimens and under minimal trauma for the specimen, which may subsequently be further processed. Likewise, the laser system can be used for marking a specimen to be resected, i.e. before resection, on an outer specimen surface accessible to a surgeon or, respectively on mucosal tissue accessible to an endoscopist (S2), wherein the specimen to be resected may particularly be marked by noninvasively applying markings still identifiable after resection, which nevertheless do not negatively affect diagnostics, thereby allowing for orienting the specimen with respect to a patient as a whole, with respect to defined anatomical axes, for marking macroscopically suspicious tissue regions, *et cetera.*

Regarding the use referenced above by S1, it has been found that a higher precision can be achieved even with easily deformable specimens, since the markings, particularly the reconstruction patterns, can be applied directly on the tissue region of diagnostic interest, such as the humorous mucosal tissue surface - which is different from previous "fiducial markings" approaches. Moreover, in contrast to the use of blockface images, the embodiments disclosed herein are not restricted in their use to individual paraffin blocks, thereby allowing for a use before embedding (which would itself introduce artefacts) and in combination with non-paraffin techniques like, for instance, cryogenic preparation of specimens (cryo-specimens). In particular, applications on "fresh" specimens, i.e. before paraffin embedding our proposed. Moreover, the embodiments disclosed herein have been found to be not restricted regarding a histological slices or, equivalently sectioning planes, which is a difference to slice-to-slice registration techniques not using markings and requiring distances between series slices or step slices to be low. Hence, the techniques disclosed herein find application in clinical and surgical specimens as well as in the context of clinical workflows dealing with large specimens and restrictions regarding the number of histological slices to be prepared - series or narrow step slices over the entire specimen will usually not be possible and the setting. Moreover, no assumption regarding a parallelity of the histological slices needs to be made, implying that reconstructions are facilitated even for large specimens, which first need to be partitioned and thereafter individually embedded and further processed. The latter is often done with clinical specimens acquired by surgery, as opposed to specimens acquired from, for instance, mice brains for academic purposes. Also, in contrast to most known techniques, a complete optimisation can be achieved, particularly a completely automatized application of the reconstruction pattern facilitating the histological reconstruction as well as of the subsequent reconstruction itself. As mentioned already an applicability is achieved independent of the mechanical stability of the specimen, which distinguishes the techniques disclosed herein from, for instance, techniques involving needle markings along a longitudinal axis of the specimen. At the same time, less tissue trauma needs to be tolerated then in known techniques, particularly no mechanical trauma (in contrast to needles, mechanical carvings, et cetera) and no or rarely any thermal trauma as compared to thermal cutting or coagulation systems, which particularly leads to the possibility to place the markings immediately in the region of interest, thereby allowing for maximum precision by a decreased distance thereto and, in turn, independence from mechanical stability.

Regarding the use referenced above by S2, a reduced risk for patients can be achieved as compared to conventional techniques like, for instance, the application of additional coagulation points. Moreover, a reduction in negative effects on diagnostic quality is achievable - coagulation immediately on a tumour boundary would negatively affect diagnose ability for the pathologists, and is therefore impractical. Also, a higher precision than with tools non-dedicated to this purpose, such as coagulation tools, needles, et cetera, can be achieved. If application of the reconstruction pattern as in S1 is combined with imaging by, e.g., OCT, any freehand- or other pattern as in S2 can be precisely positioned with respect to the S1 pattern, hence with respect to the reconstructed 3d model. By improving the positioning of the specimen with respect to the patient anatomy through an application of markings before resection, such as using a device integrated in an endoscope or in a laparoscopic instrument, an improved communication between surgeon and pathologist can be achieved. If the application of freehand- or other patterns from S2 is furthermore recorded on video, S1 and S2 combined allow for a direct and precise relation from individual positions in the perioperative video to individual positions in the histology-based 3d-model (as well as in individual tissue sections, if these have intersection points with the S2 markings). By video-to-video registration, this information can also be transferred to footage recorded before the application of the S1 markings.

In particular, combined application of S1 and S2 allows to tackle the following tasks: (i) Reconstruction of a 3d model from individual tissue sections, then transfer of that 3d model to the perspective of a pre or perioperative video. (ii) Encircling of suspicious regions by the surgeon before or during the resection, retrieval of this information by the pathologist both in individual tissue sections as well as in the 3d model, if reconstructed.

To summarise, the invention relates to A) the use of a laser system having the property that (i) in ablation of tissue is possible and that (ii) remaining tissue in the ablated tissue region is affected as little as possible, B) the purpose, to mark human tissue immediately in the diagnostically relevant region (e.g., mucosa, tumour, et cetera) without negatively affecting the quality of pathological diagnostics, C) the goal of a later orientability of the two-dimensional histological slices and the pathological findings in three-dimensional space (e.g., by generating a three-dimensional model of the specimen, or a "3D reconstruction", in short) and with respect to important body axes, and D) the application after resection (i.e., in the course of histopathological work up) and/or before resection (i.e., before or right in the course of a surgical/endoscopic intervention, integrated in corresponding tools). The invention facilitates reconstruction of a 3d model of the specimen from its histopathological tissue sections. Moreover, the invention and its parts allow to precisely relate spatial information from the different domains: (i) pre- or periinterventional video, (ii) histopathology tissue sections, (iii) 3d model derived from the histopathology tissue sections.

Although many of the embodiments described above relate to a reconstruction pattern defined with respect to a histological slicing that is still to be carried out, particularly to contour lines arising from a future histological slicing, the reconstruction pattern may actually be chosen independently from the histological slicing. In such a scenario, some reconstruction pattern may be applied to the respective surface of the specimen, and thereafter the histological slicing may be chosen to be carried out so as extract a maximum amount of information from the reconstruction pattern regarding the histological reconstruction to be carried out. In particular, a slicing direction for the histological slicing may be chosen based on the applied reconstruction pattern, possibly such that the contour lines along which the surface comprising the reconstruction pattern is decomposed in the course of the histological slicing intersect the reconstruction pattern as often as possible.

Other variations to the disclosed embodiments can be understood and effected by those skilled in the art in practicing the claimed invention, from a study of the drawings, the disclosure, and the appended claims. Such that

In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

Procedures like the providing of image data, the determining of a reconstruction pattern, the providing of a reconstruction pattern, the applying of a reconstruction pattern, the reconstructing of three-dimensional image data, et cetera, performed by one or several units or devices can be performed by any other number of units or devices. These procedures, can be implemented as program code means of a computer program and/or as dedicated hardware. However, these procedures, as well as any method following from any combination of them, are also disclosed herewith independently of how they are implemented.

Any providing unit referred to herein can be a receiving unit configured to receive respective data from a corresponding acquisition device and to provide the received data. In case the data refers to image data or a measurable quantity, for instance, the acquisition device may be an imaging device or a sensing device, for instance. However, the providing units can also be storages in which the previously acquired respective data have been stored and from which it can be retrieved for providing the same. The providing units can also be or comprise the acquisition device which acquires the respective data.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an apparatus for facilitating a three-dimensional histological reconstruction. The apparatus comprises a reconstruction pattern application device configured to apply a reconstruction pattern to a surface of a histological specimen, wherein the surface will be decomposed along slice contours arising from a predefined histological slicing to be carried out on the specimen. The applied reconstruction pattern intersects the slice contours so as to be decomposed by the histological slicing and restored upon three-dimensional histological reconstruction.

## Claims

1. An apparatus (100) for facilitating a three-dimensional histological reconstruction, wherein the apparatus comprises:
- a reconstruction pattern application device (109-115) configured to apply a reconstruction pattern to a surface of a histological specimen (10), wherein the surface will be decomposed along slice contours arising from a predefined histological slicing to be carried out on the specimen (10), wherein the applied reconstruction pattern intersects the slice contours so as to be decomposed by the histological slicing and restored upon three-dimensional histological reconstruction.

2. The apparatus (100) as defined in claim 1, wherein the apparatus comprises:
- a reconstruction pattern determining unit (111) configured to determine the reconstruction pattern to be applied to the surface of the histological specimen (10) based on the predefined histological slicing.

3. The apparatus (100) as defined in claim 2, wherein the apparatus comprises:
- an image data providing unit (110) configured to provide image data for a two-dimensional image (20) of the histological specimen (10), wherein the image (20) corresponds to a view onto the surface of the specimen (10) that will be decomposed by the histological slicing,
wherein the reconstruction pattern determining unit (111) is configured to determine the reconstruction pattern to be applied to the surface of the histological specimen (10) based further on the image data.

4. The apparatus (100) as defined in claim 3, wherein the reconstruction pattern determining unit (111) is configured to determine a boundary of the surface of the specimen (10) in the two-dimensional image (20) based on the provided image data, and to determine the reconstruction pattern based on the determined boundary.

5. The apparatus (100) as defined in any of the preceding claims, wherein the determined reconstruction pattern is a line pattern.

6. The apparatus (100) as defined in any of the preceding claims, wherein repeating elements in the determined reconstruction pattern are provided with distinguishing features.

7. The apparatus (100) as defined in any of claims 3 to 6, wherein the image data providing unit is configured to provide image data for a plurality of two-dimensional images (20) of the histological specimen (10), wherein each of the images (20) corresponds to a different view onto the surface of the specimen (10), wherein the reconstruction pattern determining unit (110) is configured to determine the reconstruction pattern with respect to each of the images (20).

8. The apparatus (100) as defined in any of the preceding claims, wherein the reconstruction pattern application device (109-115) comprises a laser (112) for applying the reconstruction pattern to the surface of the specimen (10).

9. The apparatus (100) as defined in any of the preceding claims, wherein the apparatus further comprises an imaging device, particularly an optical coherence tomography device, for imaging the applied reconstruction pattern on the surface of the specimen (10).

10. A system (200) forthree-dimensional histological reconstruction, wherein the system comprises:
- a reconstruction pattern providing unit (201) configured to provide data indicative of a reconstruction pattern that has been applied to a surface of a histological specimen (10),
- a slice image data providing unit (202) configured to provide image data for two-dimensional images (30) of histological tissue slices generated from the histological specimen (10) by a decomposition of the surface to which the reconstruction pattern has been applied along slice contours intersecting the reconstruction pattern, and
- a reconstruction unit (203) configured to reconstruct three-dimensional image data for a volumetric image of the histological specimen (10) based on the data indicative of the applied reconstruction pattern and the image data for the two-dimensional images (30) of the histological slices such that, in the volumetric image, the reconstruction pattern is restored on the surface of the histological specimen (10).

11. An apparatus (100') for marking specimen tissue to be extracted from an anatomical region (50) in a subject's body (60), wherein the apparatus comprises:
- a marking device (109', 110', 111', 112, 117) for marking the specimen tissue inside the anatomical region (50), wherein the marking device comprises:
- a pulsed infrared laser (112) for generating pulsed infrared laser light, and
- a light guide (117) for guiding the laser light from the laser (112) towards the anatomical region (50) in the subject's body (60).

12. The apparatus (100') as defined in claim 11, wherein the marking device (109', 110', 111', 112, 117) is configured to mark the specimen tissue by applying a reconstruction pattern to a surface thereof that is decomposable along slice contours arising from a histological slicing to be carried out on the specimen tissue after extraction, wherein the applied reconstruction pattern intersects the slice contours so as to be decomposed by the histological slicing and restored upon three-dimensional histological reconstruction.

13. A display device configured to display markings applied to specimen tissue to be extracted from an anatomical region (50) in a subject's body (60) in at least one of a) a two-dimensional image corresponding to a histological slice generated by a histological slicing carried out on the specimen tissue after being extracted, b) an image, particularly an optical coherence tomography image, of an applied reconstruction pattern on the specimen tissue, and c) a volumetric image of the specimen tissue generated based on a three-dimensional histological reconstruction.

14. A method (300) for facilitating three-dimensional histological reconstruction, wherein the method includes:
- applying a reconstruction pattern to a surface of a histological specimen (10), wherein the surface will be decomposed along slice contours arising from a predefined histological slicing to be carried out on the specimen (10), wherein the applied reconstruction pattern intersects the slice contours so as to be decomposed by the histological slicing and restored upon three-dimensional histological reconstruction.

15. A computer program for facilitating three-dimensional histological reconstruction, wherein the program comprises instructions causing the apparatus (100) as defined in any of claims 1 to 9 to carry out the method (300) as defined in claim 14 if the program is run on a computer controlling the apparatus.
